(19) Europäisches Patentamt
European Patent Office
Office européen des brevets

(11) Numéro de publication : **0 069 664**
**B1**

(12) **FASCICULE DE BREVET EUROPÉEN**

(45) Date de publication du fascicule du brevet : **03.04.85**

(21) Numéro de dépôt : **82401252.0**

(22) Date de dépôt : **05.07.82**

(51) Int. Cl.[4] : **C 07 D307/52**, C 07 D405/12, C 07 D409/12, A 61 K 31/34, A 61 K 31/38, A 61 K 31/44, A 61 K 31/495

(54) **Amidobenzamides, leurs sels, procédé pour leur préparation et compositions pharmaceutiques les renfermant.**

(30) Priorité : **08.07.81 FR 8113420**
**23.10.81 FR 8119967**
**10.12.81 FR 8123084**

(43) Date de publication de la demande :
**12.01.83 Bulletin 83/02**

(45) Mention de la délivrance du brevet :
**03.04.85 Bulletin 85/14**

(84) Etats contractants désignés :
**AT BE CH DE FR GB IT LI LU NL SE**

(56) Documents cités :
**FR-A- 2 471 376**
**GB-A- 2 003 471**

(73) Titulaire : **SANOFI, société anonyme**
**40, Avenue George V**
**F-75008 Paris (FR)**

(72) Inventeur : **Nisato, Dino**
**Viale Golgi, 80**
**Pavia (IT)**
Inventeur : **Boveri, Sergio**
**Corso Republica 48**
**Tortona (IT)**
Inventeur : **Bianchetti, Alberto**
**Via Corridoni 11**
**Milano (IT)**
Inventeur : **Roncucci, Romeo**
**20 rue Tournefort**
**F-75005 Paris (FR)**
Inventeur : **Carminati, Paolo**
**Via Pacini 24**
**Milano (IT)**

(74) Mandataire : **Gillard, Marie-Louise et al**
**Cabinet Beau de Loménie 55, Rue d'Amsterdam**
**F-75008 Paris (FR)**

**EP 0 069 664 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des amidobenzamides ayant une activité bloquant les récepteurs $H_2$ de l'histamine, leurs sels, un procédé pour leur préparation et des compositions pharmaceutiques les renfermant en tant qu'ingrédients actifs.

Après la subdivision des récepteurs de l'histamine en récepteurs $H_1$ (Ash et Schild, Brit. J. Pharmac. Chemother. 1966, 27, 427) et récepteurs $H_2$ (Black et al., Nature 1972, 236, 385) et la découverte que le bloc sélective des récepteurs $H_2$ provoque une inhibition de la sécrétion gastrique, de nombreux produits ont été proposés comme antagonistes des récepteurs $H_2$ de l'histamine, ci-après indiqués « $H_2$-bloqueurs ». Ainsi les composés ayant reçu les Dénominations Communes Internationales burimamide, métiamide, cimétidine, ranitidine, tiotidine, étintidine, oxmétidine ont fait l'objet d'un grand nombre de publications scientifiques et l'un d'entre eux, la cimétidine, constitue déjà un outil entre les mains du médecin pour le traitement de la maladie ulcéreuse.

Tous les produits ci-dessus sont caractérisés par la présence dans leur molécule de la structure suivante :

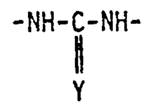

I

où Y représente un atome d'oxygène ou de soufre, ou bien un groupe N—CN ou CH—$NO_2$, ladite structure étant linéaire ou incluse dans un cycle comme dans le cas de l'oxmétidine. Les produits ci-dessus sont donc tous caractérisés par la présence de deux atomes d'azote géminaux par rapport à un atome de carbone.

La demande de brevet français publiée sous le n° 2 471 376 décrit et revendique des benzamides ayant la formule :

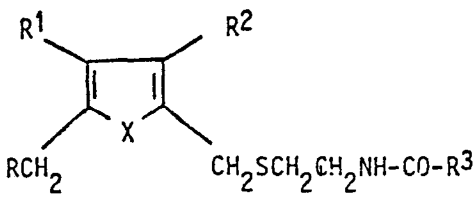

II

où R est le groupe diméthylamino ou 1-pyrrolidinyle ; $R^1$ et $R^2$ représentent chacun l'hydrogène ou un groupe alkyle en $C_1$-$C_3$ ; $R^3$ est l'hydrogène, un groupe alkyle en $C_1$-$C_3$ (substitué de manière facultative par un membre choisi dans le groupe se composant de groupes cyano, alkoxy en $C_1$-$C_3$, phényle, et d'un groupe hétérocyclique pentagonal ou hexagonal), un groupe cycloalkyle en $C_3$-$C_6$, un groupe alkényle en $C_2$-$C_5$ (substitué de manière facultative par un membre choisi dans le groupe se composant de groupes alkoxy en $C_1$-$C_3$, phényle et phénoxy), un groupe aryle en $C_6$-$C_{10}$ (substitué de manière facultative par un ou deux membres choisis dans le groupe se composant de groupes hydroxy, halogène, nitro, sulfamoyle, alkyle en $C_1$-$C_3$, alkoxy en $C_1$-$C_3$, alkanoyle en $C_1$-$C_3$, alkoxycarbonyle en $C_2$-$C_4$, dialkylamino en $C_2$-$C_4$ et alkanesulfonyle en $C_1$-$C_3$), ou un groupe hétérocyclique pentagonal ou hexagonal (éventuellement substitué par un membre choisi dans le groupe se composant de groupes oxo, halogène, alkyle en $C_1$-$C_3$ et alkoxy en $C_1$-$C_3$) et X est l'oxygène ou le soufre, ainsi qu'à leurs sels d'addition avec les acides, pharmaceutiquement acceptables.

Parmi les produits décrits dans la demande de brevet ci-dessus, le composé de formule II où R = diméthylamino, $R^1 = R^2 = H$ et $R^3$ = 4-sulfamoylphényle, à savoir la N-[2-(5-diméthylaminométhylfuran-2-ylméthylthio)éthyl]-4-sulfamoylbenzamide, sous forme d'oxalate, montre une valeur de $DE_{50}$ de 2,54 mg/kg dans l'activité d'inhibition de la sécrétion d'acide de l'estomac chez le rat.

La demande de brevet français ci-dessus, qui se réfère à des composés n'ayant pas la structure I, ne comprend, dans sa formule générale, aucun amidobenzamide, à savoir aucun benzamide substitué dans le noyau benzénique par un groupe acylamido ou sulfonylamido.

Il est également connu que des récepteurs $H_2$ de l'histamine sont situés non seulement dans la muqueuse gastrique, mais aussi dans le nœud sinusal, dans le myocarde ventriculaire et dans les vaisseaux coronariens et que les $H_2$-bloquant connus sont actifs à la fois sur les récepteurs cardiaques et gastriques. Ainsi, le blocage des récepteurs $H_2$ cardiaques peut être la cause de la bradycardie et de l'asystolie observées comme effets secondaires dans le traitement de la maladie ulcéreuse par la cimétidine (Clinica Terapeutica, 1981, *96*, 81-91, en particulier page 84).

Il est donc souhaitable de pouvoir disposer de composés présentant une dissociation entre l'activité $H_2$ bloquante gastrique et cardiaque, en faveur de la première, qui soient susceptibles de donner moins d'effets secondaires au niveau cardiaque.

On a maintenant trouvé que de nouvelles amidobenzamides, ne présentant pas la structure I ci-dessus, possèdent une bonne action antagonisant les récepteurs $H_2$ de l'histamine et que cette action se produit de préférence au niveau des récepteurs gastriques.

On a également trouvé, d'une façon surprenante, que l'activité $H_2$-bloquante ne se produit à un niveau satisfaisant que lorsque le groupe « amido » est dans la position méta du noyau phényle des benzamides.

Encore, on a trouvé que les nouvelles amidobenzamides ne montrent pas les effets secondaires caractéristiques des produits à action $H_2$-bloquante, notamment de la cimétidine, tel que l'effet anti-andromyogène.

Ainsi, la présente invention, selon un de ses aspects, a pour objet des amidobenzamides de formule :

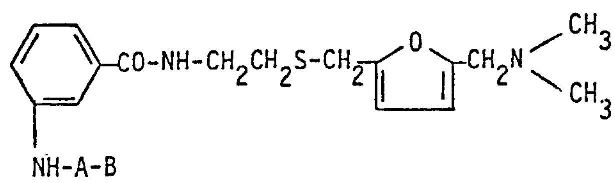

III

dans laquelle A représente un groupe CO ou $SO_2$ et B représente un groupe alkyle contenant de 1 à 6 atomes de carbone, un groupe phényle, un groupe pyridyle, un groupe pyridyle 1-oxyde, un groupe pyrazinyle ou un groupe thiényle, ainsi que leurs sels pharmaceutiquement acceptables.

Les sels pharmaceutiquement acceptables comprennent les sels non toxiques dérivés d'acides minéraux ou organiques salifiant une ou les deux fonctions basiques présentes dans la molécule des composés de formule III, tels que le chlorhydrate, le bromhydrate, le sulfate, le succinate, le tartrate, le citrate, le fumarate, le maléate, le 4,4'-méthylènebis-(3-hydroxy-2-naphtoate), ci-après désigné « pamoate », le 2-naphtalènesulfonate, ci-après désigné « napsylate », le méthanesulfonate, ci-après désigné « mésylate », le p-toluènesulfonate, ci-après désigné « tosylate », et similaires.

Selon un autre de ses aspects, la présente invention a pour objet un procédé pour la préparation des composés de formule III ci-dessus, ledit procédé étant caractérisé en ce que l'on traite la 2-(5-diméthylaminométhylfuran-2-ylméthylthio)éthylamine de formule :

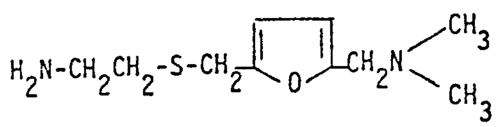

IV

avec un dérivé fonctionnel d'un acide benzoïque de formule :

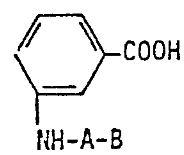

V

dans laquelle A et B ont la signification donnée ci-dessus, dans un solvant organique à une température comprise entre la température de 0 °C et la température d'ébullition du solvant employé et l'on transforme éventuellement le produit ainsi obtenu dans ses sels pharmaceutiquement acceptables.

Comme dérivé fonctionnel approprié, on peut utiliser l'anhydride, une anhydride mixte, le chlorure ou un ester activé.

Un dérivé fonctionnel préféré de l'acide de formule V ci-dessus est représenté par la formule suivante :

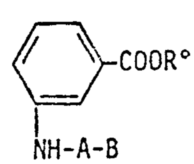

VI

dans laquelle A et B ont la signification donnée ci-dessus et R° représente un groupe nitrophényle, méthoxyphényle, trityle, benzhydryle.

La température de réaction peut varier entre 0 °C et le point d'ébullition du solvant employé, mais en général on opère à la température ambiante ou à 30-50 °C. Il peut être préférable de conduire la réaction au froid lorsqu'elle est exothermique, comme dans le cas où on utilise le chlorure en tant que dérivé fonctionnel de l'acide benzoïque de formule V.

Comme solvant de réaction on utilise de préférence un alcool, tel que le méthanol, ou un solvant halogéné, tel que le chlorure de méthylène, le dichloroéthane, le chloroforme et similaires, mais d'autres solvants organiques compatibles avec les réactifs employés, par exemple le dioxane, le tétrahydrofurane ou un hydrocarbure tel que l'hexane peuvent également être employés.

La réaction peut être conduite en présence d'un accepteur de protons, par exemple d'un carbonate alcalin ou d'une amine tertiaire, dans le cas où de l'acide chlorhydrique, ou un autre acide, se libère pendant la réaction, mais cet accepteur de protons n'est pas indispensable pour l'obtention du produit final.

La réaction est assez rapide ; en général, après 2-4 heures à la température ambiante ou à 30-50 °C la réaction est achevée et l'amidobenzamide de formule III obtenu est isolé selon les techniques conventionnelles sous forme de base libre ou d'un de ses sels.

La base libre peut être transformée dans un de ses sels pharmaceutiquement acceptables par traitement avec une solution de l'acide convenable dans un solvant organique. Si l'amidobenzamide III est isolé sous forme de sel, la base libre correspondante peut être libérée à l'aide d'un hydroxyde ou carbonate alcalin.

Les nouveaux composés de formule III de la présente invention, ainsi que leurs sels pharmaceutiquement acceptables, agissent comme antagonistes sélectifs des récepteurs $H_2$ de l'histamine en inhibant sélectivement la sécrétion gastrique au niveau des récepteurs $H_2$ gastriques avec une faible activité sur les récepteurs $H_2$ cardiaques et sont donc utiles pour le traitement de la maladie ulcéreuse.

La sélectivité de l'activité des produits de la présente invention vers les récepteurs du type $H_2$ est confirmée par l'absence d'activité du type $H_1$ dans le test de la contraction provoquée par l'histamine sur l'iléon isolé de cobaye.

L'activité antagoniste des amidobenzamides de la présente invention vers les récepteurs $H_2$ gastriques de l'histamine a été confirmée dans le test de l'activité antisécrétoire basé sur l'antagonisme pour l'hypersécrétion provoquée par l'histamine chez le rat selon la méthode de Ghosh et Schild (Brit. J. Pharmacol. 1958, 13, 54). Selon ce test, on provoque une hypersécrétion acide gastrique par infusion intraveineuse d'une dose submaximale d'histamine équivalent à 15 mcmol/kg/heure et l'on mesure la sécrétion gastrique par perfusion d'une solution physiologique à une vitesse constante dans l'estomac de l'animal.

Le tableau I montre, pour des composés représentatifs de la présente invention, désignés par leur numéro de code et pour trois produits de référence, la 2-cyano-1-méthyl-3-[2[(5-méthylimidazol-4-yl)méthylthio]éthyl]guanidine, ci-après désigné par sa Dénomination Commune Internationale « cimétidine », la N-[2-[[5-[(diméthylamino)méthyl]furfuryl]thio]déthyl]-N'-méthyl-2-nitro-1,1-éthènediamine ci-après désigné par sa Dénomination Commune Internationale « ranitidine » et le N-[2-(5-diméthylaminométhylfuran-2-ylméthylthio)-éthyl]-4-sulfamoylbenzamide, décrit dans la demande de brevet français 2 471 376 citée ci-dessus, ci-après désigné « Composé A », la dose (en mcmol/kg par voie veineuse en dose unique) qui inhibe de 50 % l'hypersécrétion gastrique provoquée par l'histamine ($DI_{50}$), qui représente l'index de l'activité $H_2$-bloquante gastrique.

Tableau I

| Composé | $DI_{50}$ (mcmol/kg) | Puissance relative (cimétidine = 1) |
|---|---|---|
| Cimétidine | 0.95 | 1.00 |
| Ranitidine | 0.25 | 3.80 |
| Composé A | 2.26 | 0.42 |
| CM 57822 | 0.98 | 0.97 |
| CM 57888 | 0.63 | 1.50 |
| SR 57912 | 0.33 | 2.88 |
| SR 57922 | 1.15 | 0.82 |
| SR 57927 A | 1.15 | 0.82 |
| SR 57933 | 1.82 | 0.52 |
| SR 57975 | 0.93 | 1.02 |
| SR 57981 A | 0.49 | 1.94 |

De ce tableau il ressort que les composés représentatifs de la présente invention sont tous plus actifs que le Composé A et que la majorité d'entre eux a une activité comparable ou supérieure à celle de la cimétidine. Deux produits, notamment, le SR 57981 et le SR 57912, sont très puissants et l'activité de ce dernier est très proche de celle de la ranitidine.

L'activité antagoniste des composés de la présente invention au niveau des récepteurs $H_2$ cardiaques de l'histamine a été évaluée dans le test de l'inhibition de l'augmentation de la fréquence induite par l'histamine sur l'oreillette droite de cobaye (D. Reinhardt et al. Agents and Actions 1974, 4 ; 217-221).

Le Tableau II montre, pour quatre produits représentatifs de la présente invention, désignés par leur numéro de code CM 57822, CM 57888, SR 57912 et SR 57922 ainsi que pour la cimétidine, la ranitidine et le Composé A, la concentration de produit sous examen qui inhibe de 50 % l'augmentation de la fréquence induite par l'histamine sur l'oreillette droite isolée de cobaye ($CI_{50}$), qui représente l'index de l'activité $H_2$-bloquante cardiaque.

Tableau II

| Composé | $CI_{50}$ mcmol/kg | Puissance relative (cimétidine = 1) |
|---|---|---|
| Cimétidine | $4\cdot10^{-7}$ | 1 |
| Ranitidine | $8\cdot10^{-8}$ | 5 |
| Composé A | $3.1\cdot10^{-7}$ | 1.3 |
| CM 57822 | $2\cdot10^{-6}$ | 0.2 |
| CM 57888 | $5.9\cdot10^{-7}$ | 0.57 |
| SR 57912 | $5\cdot10^{-6}$ | 0.08 |
| SR 57922 | $6\cdot10^{-6}$ | 0.06 |

De ce tableau il ressort que les produits représentatifs de la présente invention sont tous beaucoup moins actifs que la cimétidine comme $H_2$-bloquants cardiaques, tandis que le Composé A en est plus actif.

Les composés de la présente invention présentent donc, par rapport aux produits de l'art antérieur, une bonne dissociation entre les activités $H_2$-bloquantes cardiaque et gastrique en faveur de cette dernière.

L'effet anti-andromyogène d'un composé représentatif de la présente invention, le CM 57888, a été déterminé comparativement à la cimétidine et à la ranitidine chez des rats (9 par lot) impubères Sprague Dawley de souche CD (SD) BR (Charles River, France) castrés à l'âge de 22-23 jours et dont les annexes génitales ainsi que le Levator Ani ont été stimulés par une injection journalière de testostérone. La testostérone a été administrée par voie sous cutanée, sous forme de suspension dans le véhicule pour stéroïdes, soit à la dose de 0,2 mg/animal/jour, soit à celle de 0,4 mg/animal/jour. Pour chaque dose de testostérone, la cimétidine, la ranitidine et le CM 57888 ont été injectés simultanément par voie intrapéritonéale sous forme de solutions dans du sérum physiologique dans le cas de la ranitidine, de suspensions dans le véhicule pour stéroïdes pour les deux autres substances ; les doses utilisées ont été de 400 mg/kg/jour pour la cimétidine, de 100 mg/kg/jour pour la ranitidine et de 200 mg/kg/jour pour le CM 57888. Un lot d'animaux ne recevant que les véhicules a servi de témoins. Le traitement a duré 8 jours. 24 heures après les dernières injections, les animaux ont été sacrifiés, la prostate ventrale, les vésicules séminales et le Levator Ani immédiatement prélevés et pesés.

Il a été trouvé que les trois substances possèdent une activité anti-androgène et anti-anabolisante. Mais, aux doses utilisées, celle du CM 57888 est bien moins importante que celle des composés de référence puisque, contrairement à ces derniers, aucun effet n'a été constaté lorsque la dose de testostérone utilisée a été de 0,4 mg/animal/jour.

Cet essai montre que le produit de la présente invention est moins susceptible de donner les effets secondaires caractéristiques liés à l'action anti-andromyogène des $H_2$-bloqueurs connus.

Par rapport à leur degré d'activité, les composés de la présente invention sont peu toxiques et présentent un bon index thérapeutique.

La présente invention, selon un autre de ses aspects, se réfère ainsi à des compositions pharmaceutiques renfermant, en tant qu'ingrédients actifs, les amidobenzamides de formule III ci-dessus ainsi que leurs sels d'addition acceptables du point de vue pharmaceutique.

Les compositions pharmaceutiques à action $H_2$-bloquante de la présente invention peuvent être formulées pour l'administration orale, sublinguale, sous cutanée, intramusculaire, intraveineuse, transdermique ou rectale, en mélangeant les ingrédients actifs avec des supports pharmaceutiques classiques. Elles peuvent être administrées aux animaux et aux êtres humains dans le traitement de l'hypersécrétion gastrique et de l'ulcère peptique sous des formes unitaires d'administration.

Afin d'obtenir l'effet $H_2$-bloquant désiré, la dose de principe actif peut varier entre 1 et 100 mg par kg de poids et par jour, de préférence 10 à 50 mg par kg de poids et par jour.

Chaque dose unitaire peut contenir de 10 à 1 000 mg, de 100 à 500 de préférence, d'ingrédient actif en combinaison avec un support pharmaceutique. Cette dose unitaire peut être administrée 1 à 4 fois par jour.

Les formes unitaires d'administration comprennent les comprimés, les gélules, les poudres, les granules et les solutions ou suspensions orales et les tablettes pour l'administration sublinguale, les suppositoires ainsi que les ampoules utiles pour une administration parentérale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de sucrose ou d'autres matières appropriées ou encore on peut les traiter de telle sorte qu'ils aient une activité prolongée ou retardée et qu'ils libèrent continuellement une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant acalorique, du méthylparaben et du propylparaben comme antiseptiques, ainsi qu'un agent donnant du goût et un colorant approprié.

Les poudres ou les granulats dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une application rectale, on recourt à des suppositoires qui sont préparés avec des liants fondant à la température rectale, par exemple du beurre de cacao ou des polyéthylèneglycols.

Pour une administration orale en gouttes ou pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les exemples suivants illustrent l'invention.

## Exemple 1

On chauffe à 40 °C sous agitation pendant 2,5 heures une solution de 4,3 g (0,02 mol) de 2-(5-diméthylaminométhylfuran-2-ylméthylthio)éthylamine et de 6 g (0,02 mol) de 3-acétamidobenzoate de 4-nitrophényle dans 150 ml de méthanol puis on évapore le solvant sous pression réduite et on reprend le résidu dans 100 ml d'acide chlorhydrique N. On lave la solution acide deux fois avec 40 ml d'acétate d'éthyle et on ajuste ensuite le pH à 7,8 avec de l'hydroxyde de sodium. On extrait à fond avec de l'acétate d'éthyle, on sèche la phase organique sur du sulfate de sodium anhydre et on évapore le solvant sous pression réduite. On dissout le résidu dans 20 ml d'isopropanol et on verse la solution ainsi obtenue dans une solution de 1,7 g d'acide oxalique anhydre dans 20 ml d'isopropanol. Le produit qui précipite est filtré et cristallisé dans de l'éthanol. On obtient ainsi 6 g de 3-acétamido-N-[2-(5-dimethylaminométhylfuran-2-ylméthylthio)éthyl] benzamide oxalate, désigné par son numéro de code CM 57820 ; p. f. 123-126 °C.

De la même façon, en faisant réagir 0,02 mol de 2-(5-diméthylaminométhylfuran-2-ylméthylthio)éthylamine avec 0,02 mol de 3-propionamidobenzoate de 4-nitrophényle, avec 0,02 mol de 3-butyramidobenzoate de 4-nitrophényle et, respectivement, avec 0,02 mol de 3-triméthylacétamidobenzoate de 4-nitrophényle, on obtient :

le 3-propionamido-N-[2-(5-diméthylaminométhylfuran-2-ylméthylthio)éthyl]benzamide oxalate ; p. f. 133-135 °C ;

le 3-butyramido-N-[2-(5-dimédéthylaminométhylfuran-2-ylméthylthio)éthyl]benzamide oxalate ; p. f. 125-127 °C ; et, respectivement,

le 3-triméthylacétamido-N-[2-(5-diméthylaminométhylfuran-2-ylméthylthio)éthyl]benzamide oxalate ; p. f. 153-155 °C.

## Exemple 2

On chauffe à 45 °C sous agitation pendant 2 heures une solution de 0,02 mol de 2-(5-diméthylaminométhylfuran-2-ylméthylthio)-éthylamine et de 0,02 mol de 3-méthanesulfonamidobenzoate de 4-nitrophényle dans 150 ml de méthanol, puis on évapore le solvant sous pression réduite et on reprend le résidu dans 100 ml d'acide chlorhydrique N. On lave la solution acide à l'acétate d'éthyle et on ajuste le pH à 7,5

avec de l'hydroxyde de sodium. On extrait à fond avec de l'acétate d'éthyle, on sèche la phase organique sur du sulfate de sodium anhydre, on évapore le solvant sous pression réduite et on dissout le résidu dans 20 ml d'isopropanol chaud. On obtient ainsi le 3-méthanesulfonamido-N-[2-(5-diméthyalminométhylfuran-2-ylméthylthio)éthyl]benzamide, désigné par son numéro de code CM 57822, qui, après recristallisation de l'isopropanol, fond à 109-111 °C.

De la même façon, en faisant réagir 0,02 mol de 2-(5-diméthylaminométhylfuran-2-ylméthylthio)éthylamine avec 0,02 mol de 3-éthanesulfonamidobenzoate de 4-nitrophényle et, respectivement, avec 0,02 mol de 3-butanesulfonamidobenzoate de 4-nitrophényle, on obtient :

le 3-éthanesulfonamido-N-[2-(5-diméthylaminométhylfuran-2-ylméthylthio)éthyl]benzamide isolé sous forme d'oxalate, point de fusion 144-146 °C

le 3-butanesulfonamido-N-[2-(5-diméthylaminométhylfuran-2-ylméthylthio)éthyl]benzamide, isolé comme oxalate selon l'Exemple 1 ; SR 57981 A, p. f. 103-110 °C.

## Exemple 3

A une suspension de 0,05 mol d'acide 3-benzènesulfonamidobenzoïque et de 0,05 mol de 4-nitrophénol dans 250 ml de chlorure de méthylène anhydre on ajoute 0,05 mol de dicyclohexylcarbodiimide. On chauffe le mélange au reflux pendant 4 heures, puis on l'évapore sous pression réduite. On reprend tout le résidu dans 300 ml de méthanol et l'on ajoute à la solution ainsi obtenue 0,05 mol de 2-(5-diméthylaminométhylfuran-2-ylméthylthio)éthylamine. On chauffe le mélange réactionnel 2 heures au reflux et on l'évapore ensuite sous pression réduite jusqu'à siccité. On reprend à fond le résidu dans 150 ml d'acide chlorhydrique N et 100 ml d'acétate d'éthyle et l'on filtre. On sépare la couche organique, on ajuste à 7,8 le pH de la couche aqueuse et l'on extrait à fond cette dernière avec de l'acétate d'éthyle. On sèche les phases organiques réunies sur du sulfate de sodium anhydre et on les évapore à sec sous pression réduite. On reprend le résidu avec 40 ml d'isopropanol et l'on obtient 9,1 g de 3-benzènesulfonamido-N-[2-(5-diméthylaminométhylfuran-2-ylméthylthio)éthyl]benzamide, CM 57888, qui est cristallisée dans de l'isopropanol ; p. f. 119-121 °C.

De la même façon, on fait réagir 0,05 mol d'acide 3-(3-pyridinesulfonamido)benzoïque et, respectivement, d'acide 3-(2-thiophènesulfonamido)benzoïque avec 0,05 mol de 4-nitrophénol dans 250 ml de chlorure de méthylène anhydre en présence de 0,05 mol de dicyclohexylcarbodiimide et l'on traite l'ester activé ainsi obtenu avec 0,05 mol de 2-(5-diméthylaminométhylfuran-2-ylméthylthio)éthylamine. On obtient ainsi, respectivement,

le 3-(3-pyridinesulfonamido)-N-[2-(5-diméthylaminométhylfuran-2-ylméthylthio)éthyl]benzamide, SR 57933, sous forme de solide vitreux isolé de l'éther diéthylique ; p. f. 80-83 °C (déc.) ; et

le 3-(2-thiophènesulfonamido)-N-[2-(5-diméthylaminométhylfuran-2-ylméthylthio)éthyl]benzamide, SR 57975, cristallisé de l'acétate d'éthyle ; p. f. 113-115 °C.

## Exemple 4

En opérant comme décrit aux Exemples 1 à 3, par réaction de 0,02 mol de 2-(5-diméthylaminométhylfuran-2-ylméthylthio)éthylamine avec l'ester activé obtenu de 0,02 mol d'acide 3-benzamidobenzoïque et 0,02 mol de 4-nitrophénol en présence de dicyclohexylcarbodiimide on obtient le 3-benzamido-N-[2-(5-diméthylaminométhylfuran-2-ylméthylthio)éthyl]benzamide, SR 57916, qui, après cristallisation de l'acétate d'éthyle, fond à 112-115 °C.

De même, par réaction de la 2-(5-diméthylaminométhylfuran-2-ylméthylthio)éthylamine avec, respectivement, l'ester activé de l'acide 3-(2-thiénylcarboxamido)benzoïque, de l'acide 3-(3-pyridinecarboxamido)benzoïque, de l'acide 3-(4-pyridinecarboxamido)benzoïque, de l'acide 3-(2-pyridinecarboxamido)benzoïque et de l'acide 3-(2-pyrazinecarboxamido)benzoïque, on obtient, respectivement,

le 3-(2-thiénylcarboxamido)-N-[2-(5-diméthylaminométhylfuran-2-ylméthylthio)éthyl]benzamide, SR 57922, p. f. 116-118 °C ;

le 3-(3-pyridinecarboxamido)-N-[2-(5-diméthylaminométhylfuran-2-ylméthylthio)éthyl]benzamide dioxalate, SR 57927 A, cristallisé de l'éthanol à 95 %, p. f. 137-140 °C ;

le 3-(4-pyridinecarboxamido)-N-[2-(5-diméthylaminométhylfuran-2-ylméthylthio)éthyl]benzamide dioxalate, SR 57944 A, cristallisé de l'éthanol à 95 %, p. f. 176-178 °C ;

le 3-(2-pyridinecarboxamido)-N-[2-(5-diméthylaminométhylfuran-2-ylméthylthio)éthyl]benzamide oxalate, SR 57953 A, cristallisé de l'éthanol, p. f. 143-145 °C ; et

le 3-(2-pyrazinecarboxamido)-N-[2-(5-diméthylaminométhylfuran-2-ylméthylthio)éthyl]benzamide, SR 57939, p. f. 80-82 °C.

## Exemple 5

A une suspension de 9,5 g (0,037 mol) d'acide 3-[(3-pyridine 1-oxyde)carboxamido]benzoïque et de 5 g (0,037 mol) de 4-nitrophénol dans 400 ml de chlorure de méthylène anhydre on ajoute 7,7 g (0,037 mol) de dicyclohexylcarbodiimide. On chauffe le mélange au reflux pendant 4 heures, puis on l'évapore sous pression réduite. On reprend tout le résidu dans 300 ml de méthanol et l'on ajoute à la solution ainsi

obtenue 7,9 g (0,037 mol) de 2-(5-diméthylaminométhylfuran-2-ylméthylthio)éthylamine. On chauffe le mélange réactionnel de 2,5 heures à 40 °C et on l'évapore ensuite sous pression réduite jusqu'à siccité. On reprend à fond le résidu avec 70 ml d'eau et de l'acide chlorhydrique jusqu'à réaction nettement acide, puis on lave avec de l'acétate d'éthyle contenant 10 % d'éthanol. On sèche les phases organiques réunies sur du sulfate de sodium anhydre et on les évapore à sec sous pression réduite. On reprend le résidu avec 10 ml d'acétone et l'on obtient 0,3 g de 3-[(3-pyridine 1-oxyde)-carboxamido]-N-[2-(5-diméthylaminométhylfuran-2-ylméthylthio)éthyl]benzamide, désignée par son numéro de code SR 57912 ; p. f. 160-162 °C.

De la même façon, par réaction de 0,037 mol de 2-(5-diméthylaminométhylfuran-2-ylméthylthio)éthylamine avec l'ester activé obtenu de 0,037 mol d'acide 3-[(4-pyridine 1-oxyde)-carboxamide]benzoïque et 0,037 mol de 4-nitrophényle en présence de dicyclohexylcarbodiimide, on obtient le 3-[(3-pyridine 1-oxyde)carboxamido]-N-[2-(5-diméthylaminométhylfuran-2-ylméthylthio)éthyl]benzamide, SR 57937, cristallisé de l'isopropanol ; p. f. 145-147 °C.

## Exemple 6

A une solution de 1 g de 3-benzènesulfonamido-N-[2-(5-diméthylaminométhylfuran-2-ylméthylthio)éthyl]benzamide dans 15 ml d'éthanol on ajoute une solution de 0,3 g d'acide oxalique dans 10 ml d'éthanol. Le sel qui précipite est filtré, séché et cristallisé de 10 ml d'éthanol à 95 %. On obtient ainsi 1 g d'oxalate de 3-benzènesulfonamido-N-[2-(5-diméthylaminométhylfuran-2-ylméthylthio)éthyl]benzamide ; solide vitreux.

## Exemple 7

A une solution de 2 g d'oxalate de 3-butanesulfonamido-N-[2-(5-diméthylaminométhylfuran-2-ylméthylthio)éthyl]benzamide dans 10 ml d'eau on ajoute de l'hydroxyde de sodium jusqu'à réaction nettement basique. On extrait avec de l'acétate d'éthyle contenant 10 % d'éthanol, on sèche la phase organique sur du sulfate de sodium anhydre et on l'évapore à sec. On obtient ainsi 1,5 g de 3-butanesulfonamido-N-[2-(5-diméthylaminométhylfuran-2-ylméthylthio)éthyl]benzamide, sous forme d'huile jaunâtre.

IR : 3 500, 3 000 ; 1 640 et 1 148 $cm^1$.

Spectre $^1H$ RMN. (Solvant : DMSO-$d_6$) $\delta_{TMS}$ (ppm) : 0.83 (3H, m→t), 1.1-1.8 (4H, m), 2.11 (6H, s), 2.63 (2H, m→t), 2.9-3.6 (4H, m), 3,34 (2H, s), 3,75 (2H, s), 6.15 (2H, m), 6.2-6.8 (4H, m), 8.5 (1H, tb, disparaît par addition de $D_2O$).

## Exemple 8

Comprimés à base d'un des composés décrits aux Exemples 1 à 7, ayant la composition suivante :

| | |
|---|---|
| substance active | 100 mg |
| lactose | 70 mg |
| fécule de pommes de terre | 40 mg |
| polyvinylpyrrolidone | 8 mg |
| stéarate de magnésium | 2 mg |

On humecte le mélange de la substance active avec le lactose et la fécule de pommes de terre d'une solution alcoolique de polyvinylpyrrolidone à 15 %, on fait passer le granulé formé à travers un tamis de 1 mm, on le mélange avec le stéarate de magnésium et on forme des comprimés par compression. Poids d'un comprimé : 220 mg.

## Exemple 9

On recouvre de façon connue les comprimés fabriqués comme décrit à l'Exemple 8 d'un enrobage pour dragées consistant essentiellement en sucre et talc et on polit les dragées finies par de la cire d'abeilles. Poids d'une dragée : 300 mg.

## Exemple 10

Gélules à base d'un des composés décrits aux Exemples 1 à 7, ayant la composition suivante :

| | |
|---|---|
| principe actif | 200 mg |
| amidon de maïs | 90 mg |
| talc | 10 mg |

On mélange intimement le principe actif et les excipients et l'on introduit le mélange ainsi obtenu dans des gélules de gélatine de dimension 1. Contenu d'une gélule : 300 mg.

Exemple 11

Suppositoires à base d'un des composés décrits aux Exemples 1 à 7, ayant la composition suivante :

| | |
|---|---|
| principe actif | 300 mg |
| masse pour suppositoires (Witespol W45) | 1 450 mg |

On met en suspension la substance active finement pulvérisée dans la masse pour suppositoires à 37 °C et on verse le mélange dans des moules légèrement refroidis au préalable. Poids d'un suppositoire : 1 750 mg.

Exemple 12

Comprimés à base d'un des composés décrits aux Exemples 1 à 7, ayant la composition suivante :

| | |
|---|---|
| principe actif | 150 mg |
| cellulose microcristalline | 75 mg |
| lactose | 100 mg |
| stéarate de magnésium | 7 mg |
| talc | 18 mg |

On fait passer les poudres à travers un tamis de 0,3 mm, puis on mélange les ingrédients jusqu'à ce qu'on obtient un mélange homogène qui est comprimé et granulé. Les granules ainsi obtenus sont utilisés pour former des comprimés par compression. Poids d'un comprimé : 350 mg.

Exemple 13

En opérant comme décrit à l'exemple 12, on prépare des comprimés à base d'un des composés décrits aux exemples 1 à 7, ayant la composition suivante :

| | |
|---|---|
| principe actif | 350 mg |
| cellulose microcristalline | 100 mg |
| lactose | 125 mg |
| stéarate de magnésium | 10 mg |
| talc | 15 mg |

Poids d'un comprimé : 600 mg

Exemple 14

Comprimés à base d'un des composés décrits aux exemples 1 à 7, ayant la composition suivante :

| | |
|---|---|
| principe actif | 150 mg |
| cellulose microcristalline | 75 mg |
| talc | 15 mg |
| polyvinylpyrrolidone | 30 mg |
| silice précipitée | 25 mg |
| stéarate de magnésium | 5 mg |

On mélange intimement dans une mélangeuse-pétrisseuse tous les ingrédients, sauf le lubrifiant, pendant 15 minutes, puis on pétrit par addition graduelle d'eau. On fait passer la masse à travers un tamis de 1,25 mm. On sèche le granulé dans une étuve à ventilation forcée jusqu'à ce qu'on obtient une humidité résiduelle relativement réduite (environ 2 %). On uniformise le granulé, on ajoute le lubrifiant et on forme des comprimés par compression. Poids d'un comprimé : 300 mg.

De la même façon on prépare des comprimés contenant 250 mg de principe actif.

Exemple 15

On prépare des comprimés enrobés à base d'un des composés décrits aux exemples 1 à 7, ayant la composition suivante :

| | |
|---|---|
| principe actif | 150 mg |
| carboxyméthylamidon | 10 mg |
| cellulose microcristalline | 85 mg |
| lactose | 135 mg |

| | |
|---|---|
| huile de ricin hydrogénée | 10 mg |
| stéarate de magnésium | 5 mg |

en opérant comme décrit à l'exemple 14. On recouvre les comprimés ainsi obtenus par un revêtement ayant la composition suivante :

| | |
|---|---|
| phtalate de butyle | 0,300 mg |
| polymétacrylate de butyle de diméthylaminoéthyle | 1,850 mg |
| polyéthylèneglycol 1500 | 0,080 mg |
| silice précipitée | 0,020 mg |
| talc | 0,900 mg |
| bioxyde de titane | 1,850 mg |

dissous dans un solvant qui est éliminé par évaporation dans une étuve à ventilation forcée. Poids d'un comprimé : 400 mg.

**Revendications** (pour les Etats contractants : BE, CH, DE, FR, GB, IT, LI, LU, NL, SE)

1. Amidobenzamides de formule

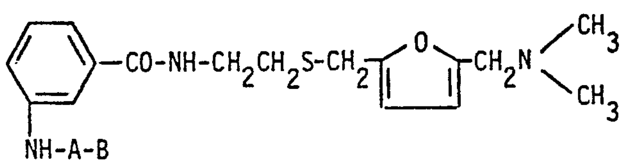

dans laquelle A représente un groupe CO ou $SO_2$ et B représente un groupe alkyle contenant de 1 à 6 atomes de carbone, un groupe phényle, un groupe pyridyle, un groupe pyridyle 1-oxyde, un groupe pyrazinyle, ou un groupe thiényle, ainsi que leurs sels pharmaceutiquement acceptables.

2. 3-méthanesulfonamido-N-[2-(5-diméthylaminométhylfuran-2-yl-méthylthio)éthyl] benzamide et ses sels pharmaceutiquement acceptables.

3. 3-éthanesulfonamido-N-[2-(5-diméthylaminométhylfuran-2-ylméthylthio)éthyl] benzamide et ses sels pharmaceutiquement acceptables.

4. 3-butanesulfonamido-N-[2-(5-diméthylaminométhylfuran-2-ylméthylthio)éthyl] benzamide et ses sels pharmaceutiquement acceptables.

5. 3-benzènesulfonamido-N-[2-(5-diméthylaminométhylfuran-2-ylméthylthio)éthyl] benzamide et ses sels pharmaceutiquement acceptables.

6. 3-(2-thiophènecarboxamido)-N-[2-(5-diméthylaminométhylfuran-2-ylméthylthio)éthyl] benzamide et ses sels pharmaceutiquement acceptables.

7. Procédé pour la préparation d'aminobenzamides selon la revendication 1, ainsi que de leurs sels pharmaceutiquement acceptables, caractérisé en ce que l'on traite la 2-(5-diméthylaminométhylfuran-2-ylméthylthio) éthylamine de formule

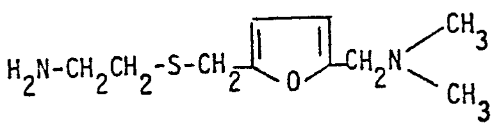

avec un dérivé fonctionnel d'un acide benzoïque de formule

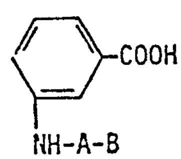

dans laquelle A et B ont la signification donnée ci-dessus, dans un solvant organique à une température comprise entre la température de 0 °C et la température d'ébullition du solvant employé et l'on transforme éventuellement le produit ainsi obtenu dans ses sels pharmaceutiquement acceptables.

8. Procédé selon la revendication 7 caractérisé en ce que comme dérivé fonctionnel de l'acide benzoïque on utilise un composé de formule

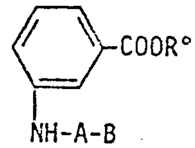

dans laquelle A et B ont la signification donnée dans la revendication 7 et R° représente un groupe nitrophényle, méthoxyphényle, trityle, benzhydryle.

9. Composition pharmaceutique à action antagoniste des récepteurs $H_2$ de l'histamine renfermant, en tant qu'ingrédient actif, un composé selon l'une des revendications 1 à 6.

10. Composition pharmaceutique selon la revendication 9, sous forme d'unité de dosage, dans laquelle chaque unité de dosage contient de 10 à 1 000 mg d'ingrédient actif en mélange avec un excipient pharmaceutique.

11. Composition pharmaceutique selon la revendication 10, contenant de 100 à 500 mg d'ingrédient actif par unité de dosage.

**Revendications** (pour l'Etat contractant AT)

1. Procédé pour la préparation d'aminobenzamides de formule

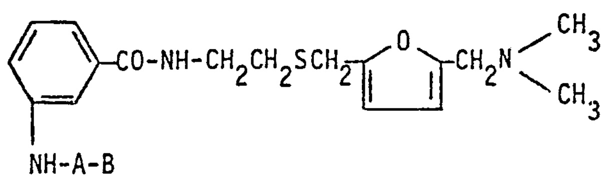

dans laquelle A représente un groupe CO ou $SO_2$ et B représente un groupe alkyle contenant de 1 à 6 atomes de carbone ou un groupe phényle, pyridyle, pyridyle 1-oxyde, pyrazinyle ou thiényle, et de ses sels pharmaceutiquement acceptables, caractérisé en ce que l'on traite la 2-(5-diméthylaminométhylfuran-2-ylméthylthio)éthylamine de formule

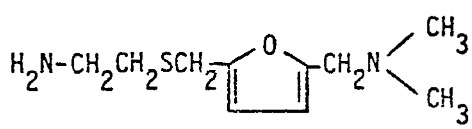

avec un dérivé fonctionnel d'un acide benzoïque de formule

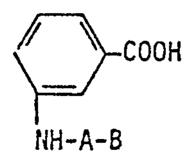

dans laquelle A et B sont tels que définis ci-dessus, dans un solvant organique à une température entre 0 °C et la température d'ébullition du solvant employé et l'on transforme éventuellement le produit ainsi obtenu dans ses sels pharmaceutiquement acceptables.

2. Procédé selon la revendication 1, caractérisé en ce que comme dérivé fonctionnel de l'acide benzoïque on utilise un composé de formule

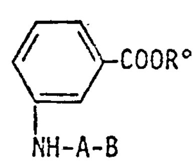

dans laquelle A et B sont tels que définis dans la revendication 1 et R° représente un groupe nitrophényle, méthoxyphényle, trityle, benzhydryle.

3. Procédé selon les revendications 1 et 2, caractérisé en ce que l'amidobenzamide est isolé sous forme d'un de ses sels.

4. Procédé selon les revendications 1 à 3, caractérisé en ce que l'on libère l'amidobenzamide base libre de ses sels à l'aide d'un hydroxyde ou d'un carbonate alcalin.

**Claims** (for the Contracting States : BE, CH, DE, FR, GB, IT, LI, LU, NL, SE)

1. Amidobenzamides of formula

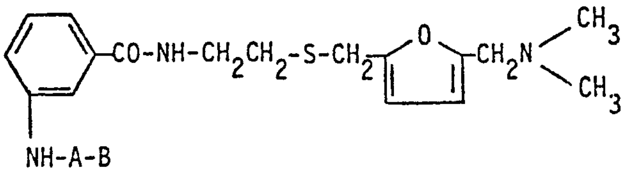

in which A represents a CO or $SO_2$ group and B represents an alkyl group of from 1 to 6 carbon atoms or a phenyl, pyridyl, pyridyl 1-oxide, pyrazinyl or thienyl group, as well as the pharmaceutically acceptable salts thereof.

2. 3-methanesulfonamido-N-[2-(5-dimethylaminomethylfuran-2-ylmethylthio-ethyl] benzamide and its pharmaceutically acceptable salts.

3. 3-ethanesulfonamido-N-[2-(5-dimethylaminomethylfuran-2-ylmethylthio)ethyl] benzamide and its pharmaceutically acceptable salts.

4. 3-butanesulfonamido-N-[2-(5-dimethylaminomethylfuran-2-ylmethylthio)ethyl] benzamide and its pharmaceutically acceptable salts.

5. 3-benzenesulfonamido-N-[2-(5-dimethylaminomethylfuran-2-ylmethylthio)ethyl] benzamide and its pharmaceutically acceptable salts.

6. 3-(2-thiophenecarboxamido)-N-[2-(5-dimethylaminomethylfuran-2-ylmethylthio)ethyl] benzamide and its pharmaceutically acceptable salts.

7. A process for the preparation of an amidobenzamide according to claim 1, as well as its pharmaceutically acceptable salts, characterized in that the 2-(5-dimethylaminomethylfuran-2-ylmethylthio)ethylamine of formula

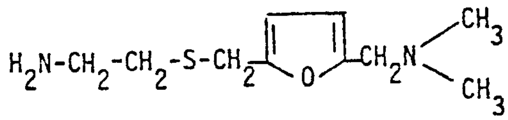

is reacted with a functional derivative of a benzoic acid of formula

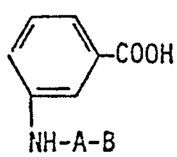

wherein A and B are as defined as above, in an organic solvent at a temperature of from 0 °C and the boiling temperature of the employed solvent and the compound thus obtained is optionally converted into its pharmaceutically acceptable salts.

8. Process according to claim 7, characterized in that as functional derivative of benzoic acid a compound of formula

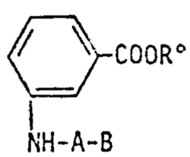

is used, in which A and B are as defined in claim 7, and R° represents a nitrophenyl, methoxyphenyl, trityl, benzhydryl group.

9. Pharmaceutical composition having histamine $H_2$ receptor blocking properties containing, as active ingredient, a compound according to any one of claims 1 to 6.

10. Pharmaceutical composition according to claim 9, in dosage unit form, in which each dosage unit contains from 10 to 1 000 mg of active ingredient in admixture with a pharmaceutical carrier.

11. Pharmaceutical composition according to claim 10, containing from 100 to 500 mg of active ingredient per dosage unit.

**Claims** (for the Contracting State AT)

1. Process for the preparation of amidobenzamides of formula

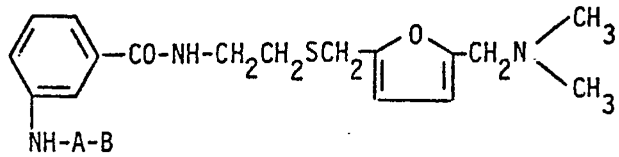

wherein A represents a CO or $SO_2$ group and B represents an alkyl group of from 1 to 6 carbon atoms or a phenyl, pyridyl, pyridyl 1-oxide, pyrazinyl or thienyl group, and its pharmaceutically acceptable salts, characterized in that the 2-(5-dimethylaminomethylfuran-2-ylmethylthio)ethylamine of formula

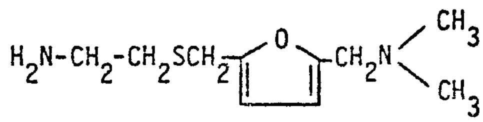

is reacted with a functional derivative of a benzoic acid of formula

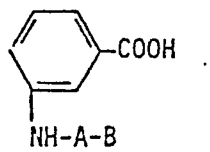

wherein A and B are as defined above, in an organic solvent at a temperature of from 0 °C and the boiling temperature of the employed solvent and optionally converting the compound thus obtained into its pharmaceutically acceptable salts.

2. Process according to claim 1, characterized in that as functional derivative of benzoic acid a compound of formula

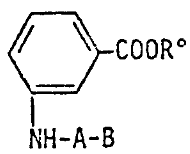

is used, wherein A and B are as defined in claim 1 and R° represents a nitrophenyl, methoxy-phenyl, trityl, benzhydryl group.

3. Process according to claim 1 and 2, characterized in that the amidobenzamide is isolated in form of a salt thereof.

4. Process according to claims 1 to 3, characterized in that the amidobenzamide free base is splitted off from its salts by means of an alcaline hydroxide or carbonate.

**Ansprüche** (für die Vertragsstaaten : BE, CH, DE, FR, GB, IT, LI, LU, NL, SE)

1. Amidobenzamide der Formel

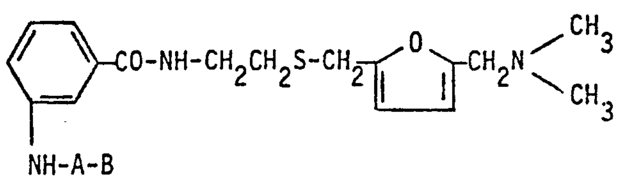

worin A eine CO- oder $SO_2$-Gruppe darstellt und B eine 1 bis 6 Kohlenstoffatome enthaltende Alkylgruppe, eine Phenylgruppe, eine Pyridylgruppe, eine Pyridyl-1-oxid-Gruppe, eine Pyrazinylgruppe oder eine Thienylgruppe bedeutet, sowie ihre pharmazeutisch akzeptablen Salze.

2. 3-Methansulfonamido-N-[2-(5-dimethylaminomethylfuran-2-yl-methylthio)-äthyl]-benzamid und seine pharmazeutisch akzeptablen Salze.

3. 3-Äthansulfonamido-N-[2-(5-dimethylaminomethylfuran-2-yl-methylthio)-äthyl]-benzamid und seine pharmazeutisch akzeptablen Salze.

4. 3-Butansulfonamido-N-[2-(5-dimethylaminomethylfuran-2-yl-methylthio)-äthyl]-benzamid und seine pharmazeutisch akzeptablen Salze.

5. 3-Benzosulfonamido-N-[2-(5-dimethylaminomethylfuran-2-yl-methylthio)-äthyl]-benzamid und seine pharmazeutisch akzeptablen Salze.

6. 3-(2-Thiophencarboxamido)-N-[2-(5-dimethylaminomethylfuran-2-yl-methylthio)-äthyl]-benzamid und seine pharmazeutisch akzeptablen Salze.

7. Verfahren zur Herstellung von Amidobenzamiden nach Anspruch 1 sowie ihren pharmazeutisch akzeptablen Salzen, dadurch gekennzeichnet, daß man 2-(5-Dimethylaminomethylfuran-2-yl-methylthio)-äthylamin der Formel

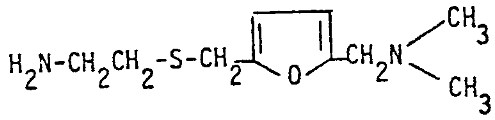

mit einem funktionellen Derivat einer Benzoesäure der Formel

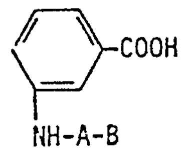

worin A und B obige Bedeutung haben, in einem organischen Lösungsmittel bei einer Temperatur zwischen 0 °C und der Kochtemperatur des verwendeten Lösungsmittels behandelt und gegebenenfalls das so erhaltene Produkt in seine pharmazeutisch akzeptablen Salze überführt.

8. Verfahren nach Anspruch 7, dadurch gekennzeichnet, daß als funktionelles Derivat der Benzoesäure eine Verbindung der Formel

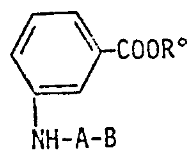

worin A und B die in Anspruch 7 angegebene Bedeutung haben und R° eine Nitrophenyl-, Methoxyphenyl-, Trityl- oder Benzhydrylgruppe darstellt.

9. Pharmazeutische Zusammensetzung mit antagonistischer Wirkung auf Histamin $H_2$-Rezeptoren, welche als aktives Ingrediens eine Verbindung nach einem der Ansprüche 1 bis 6 enthält.

10. Pharmazeutische Zusammensetzung nach Anspruch 9 in Dosiseinheitsform, wobei jede Dosiseinheit 10 bis 1 000 mg aktives Ingrediens in Mischung mit einem pharmazeutischen Exzipienten enthält.

11. Pharmazeutische Zusammensetzung nach Anspruch 10, enthaltend 100 bis 500 mg aktives Ingrediens pro Dosiseinheit.

**Ansprüche** (für den Vertragsstaat AT)

1. Verfahren zur Herstellung von Amidobenzamiden der Formel

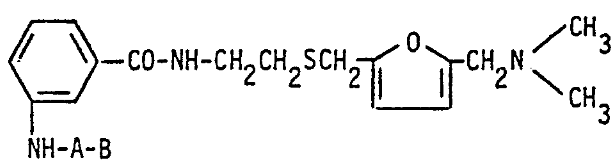

worin A eine CO- oder $SO_2$-Gruppe darstellt und B eine 1 bis 6 Kohlenstoffatome enthaltende Alkylgruppe oder eine Phenyl-, Pyridyl-, Pyridyl-1-oxid, Pyrazinyl- oder Thienylgruppe bedeutet, und deren pharmazeutisch akzeptablen Salzen, dadurch gekennzeichnet, daß man 2-(5-Dimethylaminomethylfuran-2-yl-methylthio)-äthylamin der Formel

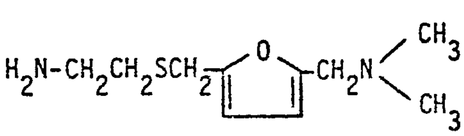

mit einem funktionellen Derivat einer Benzoesäure der Formel

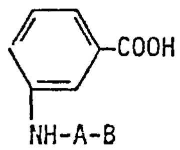

worin A und B obige Bedeutung haben in einem organischen Lösungsmittel bei einer Temperatur zwischen 0 °C und der Kochtemperatur des verwendeten Lösungsmittels behandelt und gegebenenfalls das so erhaltene Produkt in seine pharmazeutisch akzeptablen Salze überführt.

2. Verfahren nach Anspruch 1, dadurch gekennzeichnet, daß als funktionelles Derivat der Benzoesäure eine Verbindung der Formel

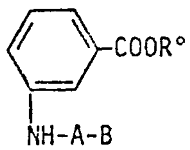

worin A und B die in Anspruch 1 angegebene Bedeutung haben und R° eine Nitrophenyl-, Methoxyphenyl-, Trityl- oder Benzhydrylgruppe darstellt.

3. Verfahren nach den Ansprüchen 1 und 2, dadurch gekennzeichnet, daß das Amidobenzamid in Form eines seiner Salze isoliert wird.

4. Verfahren nach den Ansprüchen 1 bis 3, dadurch gekennzeichnet, daß man die freie Amidobenzamidbase mit Hilfe eines Alkalihydroxids oder -carbonats aus seinen Salzen freisetzt.